(19) Europäisches Patentamt
European Patent Office
Office européen des brevets

(11) Veröffentlichungsnummer : **0 016 948**
**B1**

(12) **EUROPÄISCHE PATENTSCHRIFT**

(45) Veröffentlichungstag der Patentschrift : **03.11.82**

(51) Int. Cl.$^3$ : **C 07 C125/065**, C 07 C125/073

(21) Anmeldenummer : **80100848.3**

(22) Anmeldetag : **21.02.80**

(54) **Verfahren zur Herstellung von Urethanen.**

(30) Priorität : **02.03.79 DE 2908250**

(43) Veröffentlichungstag der Anmeldung : **15.10.80 (Patentblatt 80/21)**

(45) Bekanntmachung des Hinweises auf die Patenterteilung : **03.11.82 Patentblatt 82/44**

(84) Benannte Vertragsstaaten : **AT BE CH DE FR GB IT NL**

(56) Entgegenhaltungen :
**EP-A1-0 000 563**
**EP-A1-0 000 815**
**EP-A1-0 003 989**
**GB-A-1 486 399**

**CHEMISTRY LETTERS, 1972 Tokyo**
**K. KONDO et al. « A New Synthesis of Carbamates. The Reaction of Carbon Monoxide with Amine and Alcohol in the Co-Presence of Selenium and Triethylamine »**
**Seiten 373 bis 374**

(73) Patentinhaber : **BAYER AG**
**Zentralbereich Patente, Marken und Lizenzen**
**D-5090 Leverkusen 1, Bayerwerk (DE)**

(72) Erfinder : **Scholl, Hans-Joachim, Dr.**
**Rudolf-Sohm-Strasse 28**
**D-5000 Koeln 60 (DE)**
Erfinder : **Zenner, Armin, Dr.**
**Goethestrasse 65**
**D-4047 Dormagen 1 (DE)**

**EP 0 016 948 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Imprimerie Jouve, 18, rue St-Denis, 75001 Paris, France

# Verfahren zur Herstellung von Urethanen

Die vorliegende Erfindung betrifft ein neues Verfahren zur Herstellung von Urethanen durch Umsetzung von aromatischen Aminoverbindungen mit Alkoholen und Kohlenmonoxid in Gegenwart von Schwefel und/oder Selen und/oder Verbindungen dieser Elemente enthaltenden Katalysatorensystemen. Aus den genannten Ausgangsmaterialien zugängliche Urethane wurden bisher im allgemeinen durch Umsetzung eines aromatischen Isocyanats mit einem Alkohol gebildet, wobei das Isocyanat seinerseits durch Umsetzung von Phosgen mit dem entsprechenden primären Amin gewonnen wird, welches im allgemeinen wiederum durch Reduktion der entsprechenden Nitroverbindung erhalten worden war. Dieses herkömmliche Verfahren weist jedoch verschiedene Nachteile auf, nicht zuletzt aufgrund der Toxizität und der korrosiven Natur von Phosgen und der Bildung von Chlorwasserstoff als Nebenprodukt.

Es mangelte daher auch nicht an Versuchen, den Umweg über das hochtoxische Phosgen zu vermeiden um die Urethane direkt aus den entsprechenden Nitroverbindungen und den entsprechenden Alkoholen sowie Kohlenmonoxid herzustellen (vgl. z.B. US-PS'en 3 338 956, 3 448 140, 3 454 620, 3 467 694, 3 531 512, 3 993 685, 4 052 420, 4 052 437, GB-PS'en 1 087 896, 1 080 094, 1 246 217, 1 469 222 oder 1 472 243, die sich mit der Urethansynthese unter Verwendung von Edelmetalle der Platingruppe bzw. deren Verbindungen aufweisenden Katalysatorsystemen befassen, sowie US-PS'en 3 895 054, 3 956 360, GB-PS 1 485 108 oder GB-PS 1 486 399, die diese Urethansynthese unter Verwendung von Selen bzw. von Selenverbindungen enthaltenden Katalysatorsystemen zum Gegenstand haben).

Auch die Herstellung der Urethane aus Aminoverbindungen, den entsprechenden Alkoholen sowie Kohlenmonoxid unter Verwendung von Selen als Katalysator gehört zum vorbekannten Stand der Technik (Chemistry Letters (1972), Seiten 373-374, herausgegeben von der « Chemical Society of Japan »). Bei dem in dieser Literaturstelle beschriebenen Verfahren müssen stöchiometrische Mengen an Selen eingesetzt werden, was zu erheblichen Verlusten dieses Katalysators führt. Trotz der Verwendung dieser großen Selenmengen sind die Urethanausbeuten im Falle der Verwendung von aromatischen Aminen als Ausgangsmaterial sehr niedrig. Außerdem handelt es sich bei Selen bzw. den einzusetzenden Selenverbindungen um toxikologisch bedenkliche Substanzen, die darüber hinaus dem hergestellten Urethan einen unangenehmen Geruch verleihen.

Es war Aufgabe der vorliegenden Erfindung, ein verbessertes Verfahren zur Herstellung von Urethanen aus aromatischen Aminoverbindungen, Alkoholen und Kohlenmonoxid zur Verfügung zu stellen, bei welchem entweder ganz ohne Selen bzw. Selenverbindungen gearbeitet wird oder bei welchem die Menge des Selens bzw. der Selenverbindung ganz wesentlich reduziert werden kann.

Diese Aufgabe konnte überraschenderweise durch das nachstehend näher erläuterte erfindungsgemäße Verfahren gelöst werden.

Gegenstand der vorliegenden Erfindung ist ein Verfahren zur Herstellung von Urethanen durch Umsetzung von aromatischen Aminoverbindungen mit aliphatischen, cycloaliphatischen oder araliphatischen Alkoholen und Kohlenmonoxid, dadurch gekennzeichnet, daß man die Umsetzung in Gegenwart von

a) Schwefel und/oder Selen und/oder Verbindungen dieser Elemente,

b) aromatischen Nitroverbindungen,

c) tertiären organischen Aminen und/oder Alkalisalzen schwacher Säuren,

d) Oxidationsmitteln ausgewählt aus der Gruppe bestehend aus Sauerstoff und oxidierend wirkenden, chemisch gebundenen Sauerstoff enthaltenden anorganischen Verbindungen von Metallen der 1., 2., 5.-8. Nebengruppe des Periodensystems der Elemente und gegebenenfalls

e) Ammoniak und/oder aliphatischen, araliphatischen, cycloaliphatischen oder heterocyclischen Aminen mit mindestens einem an Aminstickstoff gebundenem Wasserstoffatom durchführt.

Ausgangsverbindungen für das erfindungsgemäße Verfahren sind

1. aromatische Aminoverbindungen, wie z.B. Aminobenzol, 4-Aminochlorbenzol, 3,4-Dichloraminobenzol, 1,3-Diaminobenzol, o-Aminotoluol, m-Aminotoluol, p-Aminotoluol, 2,4-Diaminotoluol, 2,6-Diaminotoluol, Aminonaphthaline, Aminoanthracene, Aminobiphenylene und dergleichen. Im allgemeinen weisen die erfindungsgemäß geeigneten Aminoverbindungen ein Molekulargewicht von 93 bis 300, 1 bis 3 aromatische Kerne, sowie 1 bis 3 an aromatische Kerne gebundene Aminogruppen, sowie gegebenenfalls weitere unter den Reaktionsbedingungen des erfindungsgemäßen Verfahrens inerte Substituenten auf. Zu den bevorzugten Aminoverbindungen für das erfindungsgemäße Verfahren gehören Anilin, Aminobenzol, sowie die genannten Diaminotoluole. Beliebige Gemische der genannten Aminoverbindungen können selbstverständlich ebenfalls eingesetzt werden.

2. Aliphatische, cycloaliphatische oder araliphatische Alkohole, d.h. vorzugsweise beliebige, unter den Reaktionsbedingungen ansonsten inerte organische Verbindungen mit mindestens einer aliphatischen, cycloaliphatisch oder araliphatisch gebundenen Hydroxylgruppe des Molekulargewichtsbereichs 32 bis 300. Beispiele geeigneter Alkohole sind primäre, sekundäre oder tertiäre Alkohole wie z.B. Methanol, Äthanol, n-Propanol, iso-Propanol, die verschiedenen isomeren Butanole, Cyclohexylalkohol, Benzylalkohol, Hexylalkohol, Laurylalkohol, Cetylalkohol und dergleichen. Vorzugsweise werden beim erfindungsgemäßen Verfahren einwertige Alkohole, besonders bevorzugt Äthanol und Methanol, eingesetzt.

3. Gasförmiges Kohlenmonoxid.

Das erfindungsgemäße Verfahren wird in Gegenwart der nachstehend unter a) bis d) genannten Katalysatoren bzw. an der Reaktion teilnehmenden Zusatzmittel, sowie gegebenenfalls in Gegenwart der unter e) genannten Verbindungen durchgeführt. Es handelt sich hierbei im einzelnen um a) Schwefel und/oder Selen und/oder Verbindungen dieser Elemente, b) aromatische Nitroverbindungen, c) mindestens ein tertiäres organisches Amin und/oder mindestens ein Alkalisalz einer schwachen Säure, d) bestimmte, nachstehend näher beschriebene Oxidationsmittel und gegebenenfalls e) Ammoniak und/oder mindestens ein aliphatisches, araliphatisches, cycloaliphatisches oder heterocyclisches Amin mit mindestens einem an Aminstickstoff gebundenem Wasserstoffatom.

Geeignete Komponenten a) sind beispielsweise :

aa) elementarer Schwefel in beliebiger Form, anorganische oder organische Verbindungen, vorzugsweise des zweiwertigen Schwefels wie z.B. Carbonylsulfid (COS), Schwefelwasserstoff, Alkalisulfide wie z.B. Natriumsulfid, Dimethylsulfid, Diäthylsulfid, Thiophen oder Thioharnstoff. Bevorzugt werden elementarer Schwefel, Carbonylsulfid oder solche Schwefelverbindungen, die unter den Reaktionsbedingungen des erfindungsgemäßen Verfahrens in situ Carbonylsulfid bilden, eingesetzt. Ferner geeignet sind.

ab) Selen in beliebiger Form, vorzugsweise metallisches Selen, oder anorganische Selenverbindungen wie z.B. Selendioxid oder Carbonylselenid (COSe). Prinzipiell denkbar ist auch die Verwendung von organischen Selenverbindungen, wie z.B. Dimethylselenid, Diphenylselenid und dergleichen. Unter den unter ab) genannten Cokatalysatoren ist elementares Selen besonders bevorzugt.

Elementarer Schwefel ist die besonders bevorzugte Komponente a).

Bei der Komponente b) handelt es sich um beliebige aromatisch gebundene Nitrogruppen aufweisende organische Verbindungen, die neben den genannten Gruppen, insbesondere auch noch Aminogruppen und Urethangruppen aufweisen können. Im allgemeinen handelt es sich bei der Komponente b) der erfindungsgemäß einzusetzenden Zusätze um Verbindungen bzw. Gemische von Verbindungen, welche der Formel

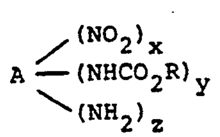

entsprechen.

In dieser Formel bedeutet

x 1 oder 2,

y 0 oder 1,

z 0 oder 1, wobei die Summe x + y + z vorzugsweise 1 oder 2 beträgt,

A einen 1-, 2- oder 3-wertigen, vorzugsweise 1- oder 2-wertigen, gegebenenfalls $C_1$-$C_4$-Alkyl-substituierten aromatischen Kohlenwasserstoffrest, der im übrigen vorzugsweise dem aromatischen Kohlenwasserstoffrest der beim erfindungsgemäßen Verfahren einzusetzenden aromatischen Aminoverbindung entspricht und

R einen aliphatichen, cycloaliphatischen oder araliphatischen Kohlenwasserstoffrest mit im allgemeinen bis zu 18 Kohlenstoffatomen, der im übrigen vorzugsweise dem Kohlenwasserstoffrest der beim erfindungsgemäßen Verfahren einzusetzenden Alkoholkomponente entspricht.

Beispiele geeigneter Komponenten b) sind Nitrobenzol, die isomeren Nitrotoluole, die isomeren Dinitrotoluole, die isomeren Aminonitrotoluole, die isomeren Nitro-alkoxy-carbonylamino-toluole, sowie beliebige Gemische der beispielhaft genannten Verbindungen. Wie bereits dargelegt, werden vorzugsweise solche Verbindungen b) eingesetzt, die bezüglich ihres aromatischen Restes der beim erfindungsgemäßen Verfahren einzusetzenden aromatischen Aminoverbindung entsprechen. Bei Verwendung von Anilin wird demgemäß beispielsweise Nitrobenzol verwendet. Dementsprechend werden bei Verwendung zweiwertiger Aminoverbindungen beispielsweise von 2,4-Diaminotoluol, die entsprechenden, zweifach substituierte Toluoylreste aufweisenden Verbindungen eingesetzt.

Bei der Komponente c) handelt es sich um tertiäre Aminogruppen aufweisende organische Basen wie z.B. tertiäre aliphatische Amine mit insgesamt 3 bis 20 Kohlenstoffatomen wie Trimethylamin, Triäthylamin, N,N-Dimethyl-octadecylamin oder Trihexylamin, heterocyclische tertiäre Amine wie z.B. Pyridin oder 2 tert. Aminogruppen aufweisende Amine wie z.B. Diazabicyclo[2.2.2]-octan (Triäthylendiamin), oder bicyclischen Amidine der Formel

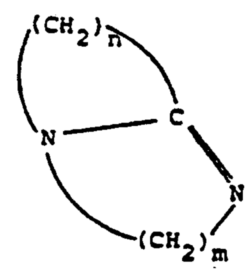

in welcher

n für eine ganze Zahl von 3 bis 5 und

m für eine ganze Zahl von 2 bis 4 stehen.

Neben oder anstelle der genannten tertiären Amine können als Komponente c) auch basisch wirkende Alkalisalze schwacher Säuren, insbesondere Alkalicarboxylate wie Natriumacetat, Kaliumacetat, Natriumbenzoat oder Alkalisalze schwacher anorganischer Säuren, wie z.B. Natriumborat oder Natriumcarbonat, verwendet

werden. Zu den bevorzugten Komponenten c) gehören 1,5-Diazabicyclo[4.3.0]-non-5-en und 1,8-Diazabicyclo[5.4.0]-undecen-7. Bevorzugt kommt weiterhin Triäthylendiamin, insbesondere in Kombination mit Salzen der Formel

MeX

zum Einsatz, wobei
Me für ein Alkalimetall-Kation und
X für ein Jodid-, Cyanat- oder Rhodanid-Anion stehen.

Besonders bevorzugt sind Kaliumacetat und Natriumacetat.

Bei Verwendung derartiger Kombinationen werden die letztgenannten Salze im allgemeinen in Mengen von 10 bis 60 Mol-%, vorzugsweise von 5 bis 40 Mol-%, bezogen auf die eingesetzte Aminoverbindung, verwendet.

Bei den Oxidationsmitteln d) handelt es sich entweder um elementaren Sauerstoff bzw. ein Sauerstoff enthaltendes Gas wie z.B. Luft und/oder um oxidierend wirkenden chemisch gebundenen Sauerstoff aufweisende anorganische Verbindungen von Metallen. Bei den letztgenannten Verbindungen handelt es sich insbesondere um die entsprechenden Oxide. Es kommen vorzugsweise die entsprechenden Metallverbindungen der Elemente der 1., 2. sowie 5. bis 8. Nebengruppe des Periodensystems in Betracht. Besonders bevorzugt werden jedoch die entsprechenden Verbindungen der Elemente der 5. und 6. Nebengruppe sowie die entsprechenden Verbindungen von Mangan, Eisen, Kobalt und Nickel eingesetzt. Beispiele geeigneter Oxidationsmittel sind Eisen-II-oxid, Eisen-III-oxid, aus diesen letztgenannten Eisenoxiden bestehende Mischoxide, Vanadium-V-oxid, Mangan-IV-oxid, Molybdän-VI-oxid, Nickel-II-oxid, Kobalt-II-oxid, Mischoxide des 3- bis 6-wertigen Chroms, sowie beliebige Gemische der beispielhaft genannten Oxide. Zu den besonders bevorzugten Oxidationsmitteln gehört Eisen-III-oxid. Ganz besonders bevorzugt sind Eisen, Vanadium und/oder Molybdän enthaltende Mischoxide.

Bei der gegebenenfalls zuzusetzenden Komponente e) handelt es sich um Ammoniak und/oder um beliebige aliphatische, araliphatische, cycloaliphatische oder heterocyclische Amine mit mindestens einem an Aminstickstoff gebundenen Wasserstoffatom. Bevorzugt werden als Komponente e) sekundäre Amine der Formel

$$R_1NH—R_2$$

eingesetzt, wobei
$R_1$ und $R_2$ für gleiche oder verschiedene Reste stehen und Alkylreste mit 1 bis 6 Kohlenstoffatomen, Cycloalkylreste mit 5 bis 6 Kohlenstoffatomen bedeuten, oder wobei $R_1$ und $R_2$ zusammen mit dem sekundären Aminstickstoffatom einen gegebenenfalls auch Sauerstoff als zweites Heteroatom enthaltenden, vorzugsweise 6-gliedrigen heterocyclischen Ring bilden.

Zu den bevorzugten Aminen gehören Dimethylamin, Diäthylamin, Dipropylamin, Dibutylamin, Methyl-hexyl-amin, Dihexylamin oder Morpholin. Dibutylamin oder Morpholin sind besonders bevorzugt.

Bei der Durchführung des erfindungsgemäßen Verfahrens kommen die Reaktionspartner im allgemeinen in solchen Mengen zum Einsatz, daß für jede Aminogruppe der als Ausgangsmaterial eingesetzten aromatischen Aminoverbindung zwischen 1 bis 50, vorzugsweise zwischen 5 bis 30 Hydroxylgruppen der Alkoholkomponente vorliegen. Das Kohlenmonoxid wird im allgemeinen im Überschuß eingesetzt, da stets in Kohlenmonoxid-Atmosphäre gearbeitet wird ; die gegebenenfalls den erfindungsgemäßen Anteil Sauerstoff aufweist.

Die Komponente a), welche auch auf einen geeigneten Träger wie Kohlenstoff, Aluminiumoxid, Siliciumdioxid, Diatomeenerde, aktivierter Ton, Zeolith, Molekularsieben, Bariumsulfat, Kalziumcarbonat, Ionenaustauscherharzen und ähnlichen Materialien aufgebracht werden kann, wird im Falle der Verwendung von Schwefel oder Schwefelverbindungen in einer solchen Menge eingesetzt, die 0,1 bis 40 Gew.-%, vorzugsweise 5 bis 25 Gew.-%, Schwefel in freier oder gebundener Form, bezogen auf die als Ausgangsmaterial eingesetzte Aminoverbindungen entspricht und im Falle der Verwendung von Selen oder von Selenverbindungen in einer Menge, die 0,01 bis 15 Gew.-%, vorzugsweise 0,1 bis 10,0 Gew.-% an freiem oder gebundenem Selen, bezogen auf die Aminoverbindungen, entspricht.

Die an der Reaktion teilnehmende Komponente b) liegt im Reaktionsgemisch im allgemeinen in einer Menge von 10 bis 70, vorzugsweise 25 bis 60 Mol-%, bezogen auf die Gesamtzahl der Mole der als Ausgangsverbindung eingesetzten aromatischen Amine und der Komponente b), vor.

Die Komponente c) liegt im Reaktionsgemisch im allgemeinen in einer Menge von 5 bis 60, vorzugsweise 5 bis 40 Mol-%, bezogen auf als Ausgangsmaterial verwendete Aminoverbindung vor, wobei sich diese Angaben auf die Gesamtmenge an basischen Verbindungen, nicht jedoch auf die gegebenenfalls mitzuverwendenden Salze der Formel

MeX

beziehen.

Die Komponente d), d.h. das Oxidationsmittel wird bei Verwendung von Sauerstoff bzw. von Sauerstoff enthaltendem Gas so gewählt, daß der Sauerstoffanteil 0,01 bis 6,0 Vol.-%, vorzugsweise 0,1 bis 2 Vol.-%, bezogen auf eingesetztes Kohlenmonoxid, beträgt. Aus Sicherheitsgründen sollten 6,0 Vol.-% nicht überschritten werden. Im Falle der Verwendung von oxidierend wirkenden Metallverbindungen werden diese im allgemeinen in Mengen von 0,1 bis 100 Gew.-%, vorzugsweise 5 bis 40 Gew.-%, bezogen auf die eingesetzte Aminoverbindung, verwendet.

Die gegebenenfalls mitzuverwendende Komponente e) liegt im Reaktionsgemisch im allgemeinen in einer Menge von 0,01 bis 20 Mol-%, vorzugsweise 0,1 bis 15 Mol-%, bezogen auf als Ausgangsmaterial eingesetzte Aminoverbindung, vor.

Das erfindungsgemäße Verfahren kann in Abwesenheit eines Lösungsmittels erfolgen, da der Alkohol als Lösungsmittel dient, jedoch kann auch ein Lösungsmittel verwendet werden. Beispiele für solche Lösungsmittel umfassen aromatische Lösungsmittel, wie Benzol, Toluol, Xylol usw., Nitrile, wie Acetonitril, Benzonitril, usw., Sulfone, wie Sulfolan, aliphatische halogenierte Kohlenwasserstoffe, wie 1,1,2-Trichlor-1,2,2-trifluoräthan, aromatische halogenierte Kohlenwasserstoffe, wie Monochlorbenzol, Dichlorbenzol, Trichlorbenzol usw., Ketone, Ester und andere Lösungsmittel, wie Tetrahydrofuran, 1,4-Dioxan, 1,2-Dimethoxyäthan und ähnliches.

Die Reihenfolge der Zugabe der Ausgangsmaterialien und der angeführten Komponenten a) bis e) ist nicht beschränkt und kann wahlfrei je nach der Art der verwendeten Apparatur verändert werden. Beispielsweise wird eine Ausgangsmischung aus Alkohol, organische Aminoverbindung und den Komponenten a) bis e) in einem geeigneten druckbeständigen Reaktor, wie ein Autoklav, eingeführt, worauf weiter Kohlenmonoxid unter Druck eingebracht wird, wonach unter Erwärmung gerührt wird, bis die Urethanbildungsreaktion beendet ist. Kohlenmonoxid und gegebenenfalls das Oxidationsmittel kann in den Reaktor entweder halbkontinuierlich oder kontinuierlich eingeleitet werden, während das beim Fortschreiten der Reaktion gebildete Kohlendioxid abgetrennt wird. Die Reaktion kann sowohl ansatzweise als auch halbkontinuierlich oder kontinuierlich durchgeführt werden. Das nach Beendigung der Reaktion im Überschuß anwesende Kohlenmonoxid kann durch Rezirkulation erneut verwendet werden.

Die Reaktionstemperatur wird im allgemeinen im Bereich von 80 bis 220 °C, vorzugsweise 120 bis 200 °C, gehalten. Obwohl die Reaktion bei höheren Reaktionstemperaturen schneller abläuft, besteht bei Temperaturen oberhalb 220 °C die Neigung zu einer thermischen Zersetzung, wodurch die Ausbeute an Urethanprodukt verringert wird. Der Reaktionsdruck, d.h. der anfängliche Kohlenmonoxiddruck vor Beginn des Aufheizens, liegt im allgemeinen im Bereich von 10 bis 300 bar, vorzugsweise 20 bis 150 bar. Die Reaktionszeit hängt von der Natur und Art der verwendeten Aminoverbindung, der Reaktionstemperatur, dem Reaktionsdruck, der Art und Menge der Komponenten a) bis e) und der Art der Apparatur ab, jedoch liegt sie im allgemeinen im Bereich von 5 Minuten bis 6 Stunden. Nach Beendigung der Reaktion wird die Reaktionsmischung der Abkühlung überlassen bzw. abgekühlt. Nach dem Ablassen des eingefüllten Gases wird die Reaktionsmischung einer bekannten Abtrennung, wie Filtration, Destillation oder einer anderen geeigneten Methode, unterworfen, um das gebildete Urethan abzutrennen.

Die nach Abtrennung des Urethans zurückbleibenden Reaktionsanteile enthalten die Komponenten a) bis e) sowie nicht abgetrennte Urethanrestanteile. Die Wiederverwendung dieser Rückstände ist insbesondere bei kontinuierlicher Verfahrensweise von Vorteil.

Bei der Durchführung des erfindungsgemäßen Verfahrens sollte auf Wasserausschluß geachtet werden, da eine teilweise hydrolytische Spaltung der erfindungsgemäßen Verfahrensprodukte bei Anwesenheit von Wasser nicht ausgeschlossen werden kann.

Der erfindungswesentliche Punkt ist beim erfindungsgemäßen Verfahren in erster Linie in der gleichzeitigen Mitverwendung der Komponenten b), d) und gegebenenfalls e) zu sehen, die in Kombination mit der Komponente c) selbst dann einen ausgezeichneten Umsatz ermöglichen, wenn als Komponente a) Selen bzw. Selenverbindungen in ganz drastisch reduzierten Mengen eingesetzt wird, bzw. wenn auf die Verwendung von Selen bzw. von Selenverbindung überhaupt verzichtet und an ihrer Stelle Schwefel bzw. Schwefelverbindungen zum Einsatz gelangen. Im Augenblick steht für diese überraschende Wirkung der erfindungsgemäßen Zusätze keine plausible Erklärung zur Verfügung.

Die erfindungsgemäßen Verfahrensprodukte stellen wertvolle Zwischenprodukte zur Herstellung von Schädlingsbekämpfungsmitteln oder zur Herstellung von Polyurethanen dar. Insbesondere eignen sich die erfindungsgemäßen Verfahrensprodukte als Ausgangsmaterialien zur Herstellung der entsprechenden Isocyanate bzw. Polyisocyanate durch an sich bekannte Abspaltung der Alkoholkomponente.

Die nachfolgenden Beispiele veranschaulichen die Erfindung ohne sie jedoch einzuschränken. In den Beispielen wurden alle Reaktionen in einem mit einem Rührer versehenen Autoklaven aus rostfreiem Stahl (V4A) durchgeführt. Die in den Beispielen angegebenen Mengenangaben wurden jeweils aus den Ergebnissen der Gaschromatographie und Flüssigkeitschromatographie errechnet, in ausgewählten Beispielen wurden die erfindungsgemäßen Verbindungen zusätzlich isoliert.

Beispiel 1

9,26 g Anilin, 2,45 g Diazabicyclo[2.2.2]-octan, 1,94 g Kaliumrhodanid, 0,14 g metallisches Selen, 8,6 g Nitrobenzol und 140 g abs. Äthanol wurden in einen 0,7 l Autoklaven eingebracht. Der Autoklav wurde 5 Minuten mit trockener Luft gespült, anschließend wurde Kohlenmonoxid in den Autoklaven unter Druck eingeleitet, bis der Anfangsdruck 50 bar bei Raumtemperatur erreichte. Unter Rühren wurde das Reaktionssystem auf 170 °C aufgeheizt und 1 Stunde bei 170 °C nachgerührt. Man ließ auf Raumtemperatur abkühlen, entspannte die Reaktionslösung, spülte mit Stickstoff und trennte von festen Anteilen durch Filtration. Das erhaltene Filtrat wurde einer gaschromatographischen Analyse unterzogen, nach der sich im Filtrat 24,3 g Äthyl-N-phenylcarbamat und 1,5 g Anilin befanden. Eine Gasanalyse vor der Reaktion zeigte die Anwesenheit von 0,9 Vol.-% $O_2$ und nach der Reaktion von 0,2 Vol.-% $O_2$ in Gasraum des Reaktionsgefäßes.

Vergleichsbeispiel

Beispiel 1 wurde ohne Luftspülung wiederholt mit der Maßgabe, daß das Reaktionssystem vor Reaktionsbeginn mit Stickstoff und nachfolgend mit Kohlenmonoxid gespült wurde. Nach entsprechendem Aufdrücken von 50 bar Kohlenmonoxid wurde wie in Beispiel 1 verfahren. Das Filtrat enthielt nur 14,8 g Äthyl-N-phenylcarbamat und 6,1 g Anilin.

Beispiel 2

29,9 g Anilin, 0,4 g Selen, 19,7 g Nitrobenzol, 2 g Kaliumacetat, 4 g eines Metalloxidgemisches aus Eisen-III-oxid und Vanadinpentoxid im Gewichtsverhältnis 11 : 1 und 300 g abs. Methanol wurden in einen 1,3 l Autoklaven eingebracht. Der Autoklav wurde mit Stickstoff und nachfolgend mit Kohlenmonoxid gespült. Anschließend wurde Kohlenmonoxid in den Autoklaven unter Druck eingeleitet, bis der Anfangsdruck 120 bar erreichte. Unter Rühren wurde auf 160 °C aufgeheizt und 2 Stunden bei 160 °C nachgerührt. Nach gaschromatographischer Analyse befanden sich in der flüssigen Phase 54,8 g Methyl-N-phenylcarbamat und 8,9 g Anilin.

Im folgenden ist beispielhaft die Aufarbeitung des Ansatzes aus Beispiel 2 zur Isolierung des Methyl-N-phenylcarbamats beschrieben :

Feste Bestandteile wurden abfiltriert und die Lösung zur Entfernung des Methanols destilliert. Der Rückstand wurde in 200 g Toluol aufgenommen und der Toluol-Extrakt filtriert. Das Filtrat wurde nachfolgend vom Toluol befreit und der Destillation unterworfen :

1. Fraktion : Anilin, 8,4 g, $Kp_{0,2}$ : 65-70 °C
2. Fraktion : Methyl-N-phenylcarbamat, 54,7 g (97 % rein), $Kp_{0,2}$ : 78-80 °C.

Beispiel 3

Beispiel 2 wurde mit 22,4 g Anilin und 29,6 g Nitrobenzol wiederholt. Die gaschromatographische Analyse ergab 65,1 g Methyl-N-phenylcarbamat und 3 g Anilin.

Die Aufarbeitung des Ansatzes aus Beispiel 3 erfolgte gemäß Beispiel 2 mit der zusätzlichen Maßnahme, daß der filtrierte Toluol-Extrakt mit wäßriger Natronlauge (2,5 %) und Wasser ausgeschüttelt wurde. Die wäßrige Phase wurde nachfolgend abgetrennt, die organische Phase vom Toluol befreit und der Destillation unterworfen ;

1. Fraktion : Anilin, 2,0 g, $Kp_{0,2}$ : 65-68 °C
2. Fraktion : Methyl-N-phenylcarbamat, 64,8 g (98 % rein), $Kp_{0,2}$ : 78-81 °C.

Beispiel 4

22,4 g Anilin, 0,4 g Selen, 29,6 g Nitrobenzol, 2 g Kaliumacetat und 300 g abs. Äthanol wurden in einen 1,3 l Autoklaven eingebracht. Der Autoklav wurde 5 Minuten mit trockener Luft gespült, anschließend wurde Kohlenmonoxid in den Autoklaven unter Druck eingeleitet, bis der Anfangsdruck 120 bar bei Raumtemperatur erreichte. Unter Rühren wurde auf 150 °C aufgeheizt und 2 Stunden bei 150 °C nachgerührt. Nach gaschromatographischer Analyse befanden sich in der flüssigen Phase 64,4 g Äthyl-N-phenylcarbamat und 4,5 g Anilin.

Beispiel 5

33,6 g Anilin, 0,4 g Selen, 14,8 g Nitrobenzol, 2 g Kaliumacetat, 4 g Metalloxidgemisch gemäß Beispiel 2 und 300 g abs. Äthanol wurden in einen 1,3 l Autoklaven eingebracht. Die Reaktionsdurchführung erfolgte gemäß Beispiel 2 bei 150 °C, 3 Stunden. Nach gaschromatographischer Analyse befanden sich in der flüssigen Phase 39,8 g Äthyl-N-phenylcarbamat und 18,7 g Anilin.

Beispiel 6

7,5 g Anilin, 0,2 g Selen, 19,7 g Nitrobenzol, 1 g Kaliumacetat, 2 g Metalloxidgemisch gemäß Beispiel 2 und 150 g abs. Äthanol werden entsprechend Beispiel 5 150 Minuten in einem 0,7 l Autoklav zur Reaktion gebracht. Die gaschromatographische Analyse ergab 32,6 g Äthyl-N-phenylcarbamat und 2,4 g Anilin.

Beispiel 7

22,0 g 2,4-Diaminotoluol, 1,0 g Selen, 32,7 g 2,4-Dinitrotoluol, 4 g Kaliumacetat, 6 g Metalloxidgemisch gemäß Beispiel 2 und 300 g abs. Methanol wurden in einen 1,3 l Autoklaven eingebracht. Die Luft im Autoklaven wurde durch Stickstoffgas und dann durch Kohlenoxid ersetzt. Anschließend wurde Kohlenoxid unter Druck in den Autoklaven geleitet bis der Anfangsdruck von 120 bar bei Raumtemperatur erreicht war. Das Reaktionssystem wurde unter Rühren aufgeheizt und 3 Stunden auf 160 °C gehalten. Die nach der Flüssigkeitschromatographie vorgenommene Analyse des vom Selen und Metalloxidgemisch abgetrennten Reaktionsgemisches, das aus Löslichkeitsgründen zusätzlich mit 200 g Methanol versetz wurde, ergab 67,3 g 2,4-Dimethoxycarbonylamino-toluol und 1,0 g 2,4-Diaminotoluol. Langsames Abkühlen auf − 10 °C erbrachte nach Filtration und Trocknung des gebildeten Kristallbreis 62,5 g 2,4-Dimethoxycarbonyl-amino-toluol vom Fp. 166-168 °C.

Beispiel 8

11,0 g 2,4-Diaminotoluol, 1,0 g Selen, 16,4 g

2,4-Dinitrotoluol, 2 g Kaliumacetat, 3 g Metalloxidgemisch gemäß Beispiel 2 und 250 g abs. Äthanol wurden, wie in Beispiel 2 beschrieben, umgesetzt. Die Flüssigkeitschromatographie-Analyse ergab 25,4 g 2,4-Diethoxycarbonyl-aminotoluol und 1,3 g 2,4-Diaminotoluol.

Beispiel 9

14,7 g 2,4-Diaminotoluol, 1,0 g Selen, 10,9 g 2,4-Dinitrotoluol, 2 g Kaliumacetat, 200 g abs. Methanol und 3 g Metalloxidgemisch gemäß Beispiel 2 wurden, wie in diesem Beispiel beschrieben, in einem 0,7 l Autoklav umgesetzt. Die Analyse ergab 19,5 g 2,4-Dimethoxycarbonylaminotoluol und 3,3 g 2,4-Diaminotoluol.

Beispiel 10

Beispiel 9 wurde mit 250 g abs. Äthanol statt Methanol wiederholt. Die Flüssigkeitschromatographie-Analyse ergab 17,9 g 2,4-Diäthoxycarbonylaminotoluol und 2,1 g 2,4-Diaminotoluol.

Beispiel 11

15,3 g 4-Chloranilin, 2 g Schwefel, 18,9 g 4-Nitrochlorbenzol, 1 g Kaliumacetat, 2 g Metalloxidgemisch gemäß Beispiel 2, 0,6 g Di-n-butylamin und 150 g Äthanol wurden, wie in Beispiel 2 ausgeführt, in einem 0,7 l Autoklav zur Reaktion gebracht. Nach gaschromatographischer Analyse befanden sich in der flüssigen Phase 24,5 g Äthyl-N-(4-chlor-phenyl)-carbamat und 7,8 g 4-Chloranilin.

Beispiel 12

14,9 g Anilin, 0,5 g Selen, 9,8 g Nitrobenzol, 1 g 1,8-Diazabicyclo[5.4.0]-undecen-7, 3 g Metalloxidgemisch gemäß Beispiel 2 und 150 g abs. Methanol wurden gemäß Beispiel 11 1 Stunde bei 170 °C zur Reaktion gebracht. Die gaschromatographische Analyse ergab 19,9 g Methyl-N-phenylcarbamat und 6,5 g Anilin.

Beispiel 13

16,8 g Anilin, 0,2 g Selen, 7,4 g Nitrobenzol, 1 g Kaliumacetat, 3 g Metalloxidgemisch gemäß Beispiel 2 und 150 g abs. Methanol wurden gemäß Beispiel 11 3 Stunden bei 150 °C zur Reaktion gebracht. Die gaschromatographische Analyse ergab 20,3 g Methyl-N-phenylcarbamat und 5,9 g Anilin.

Beispiel 14

14,9 g Anilin, 2 g Schwefel, 9,9 g Nitrobenzol, 1 g Kaliumacetat, 0,6 g Di-n-butylamin, 2 g Metalloxidgemisch gemäß Beispiel 2 und 150 g abs. Methanol wurden gemäß Beispiel 11 2 Stunden bei 170 °C zur Reaktion gebracht. Die gaschromatographische Analyse erbrachte 15,8 g Methyl-N-phenylcarbamat und 12,5 g Anilin.

Vergleichsbeispiel

Beispiel 14 wurde ohne Metalloxidgemisch und ohne Di-n-butylamin wiederholt. Die gaschromatographische Analyse ergab 1,8 g Methyl-N-phenyl-carbamat und 11,6 g Anilin.

Beispiel 15

Beispiel 3 wurde unter Zusatz von 1,3 g Di-n-butylamin mit 4 g Schwefel anstatt Selen und mit 300 g abs. Äthanol anstatt Methanol wiederholt. Die gaschromatographische Analyse ergab 40,3 g Äthyl-N-phenylcarbamat und 17,6 g Anilin.

**Ansprüche**

1. Verfahren zur Herstellung von Urethanen durch Umsetzung von aromatischen Aminoverbindungen mit aliphatischen, cycloaliphatischen oder araliphatischen Alkoholen und Kohlenmonoxid, dadurch gekennzeichnet, daß man die Umsetzung in Gegenwart von
a) Schwefel und/oder Selen und/oder Verbindungen dieser Elemente,
b) aromatischen Nitroverbindungen,
c) tertiären organischen Aminen und/oder Alkalisalzen schwacher Säuren,
d) Oxidationsmitteln ausgewählt aus der Gruppe bestehend aus Sauerstoff und oxidierend wirkenden, chemisch gebundenen Sauerstoff enthaltenden anorganischen Verbindungen von Metallen der 1., 2., 5.-8. Nebengruppe des Periodensystems der Elemente und gegebenenfalls
e) Ammoniak und/oder aliphatischen, araliphatischen, cycloaliphatischen oder heterocyclischen Aminen mit mindestens einem an Aminstickstoff gebundenem Wasserstoffatomdurchführt.

2. Verfahren gemäß Anspruch 1, dadurch gekennzeichnet, daß man als aromatische Aminoverbindung Anilin oder ein Diaminotoluol und als Alkohol Äthylalkohol oder Methylalkohol verwendet.

3. Verfahren gemäß Anspruch 1 und 2, dadurch gekennzeichnet, daß man als Komponente a) elementaren Schwefel verwendet.

4. Verfahren gemäß Anspruch 1 bis 3, dadurch gekennzeichnet, daß man als Komponente b) Nitrobenzol oder ein Dinitrotoluol verwendet.

5. Verfahren gemäß Anspruch 1 bis 4, dadurch gekennzeichnet, daß man als Komponente c) tert.-organische Amine und/oder Alkalisalze schwacher Säuren ausgewählt aus der Gruppe bestehend aus Triäthylendiamin, Kaliumacetat und Natriumacetat verwendet.

6. Verfahren gemäß Anspruch 1 bis 4, dadurch gekennzeichnet, daß man als Komponente c) ein tert.-organisches Amin in Kombination mit einem Salz der Formel

MeX

verwendet, wobei

Me für ein Alkalimetall-Kation und

X für ein Jodid-, Cyanat- oder Rhodanid-Anion steht.

7. Verfahren gemäß Anspruch 1 bis 6, dadurch gekennzeichnet, daß man als Komponente e) sekundäre Amine verwendet.

## Claims

1. A process for the production of urethanes by reacting aromatic amino compounds with aliphatic, cycloaliphatic or araliphatic alcohols and carbon monoxide, characterised in that the reaction is carried out in the presence of :

a) sulphur and/or selenium and/or compounds of these elements,

b) aromatic nitro compounds,

c) tertiary organic amines and/or alkali metal salts of weak acids,

d) oxidising agents selected from the group comprising oxygen and oxidising inorganic compounds, containing chemically bound oxygen, of metals of the 1st, 2nd, 5th to 8th Secondary Groups of the Periodic System of elements and, optionally,

e) ammonia and/or aliphatic, araliphatic, cycloaliphatic or heterocyclic amines containing at least one hydrogen atom attached to an amine nitrogen.

2. A process according to Claim 1, characterised in that aniline or a diaminotoluene is used as the aromatic amino compound and ethyl alcohol or methyl alcohol is used as the alcohol.

3. A process according to Claims 1 and 2, characterised in that elemental sulphur is used as component a).

4. A process according to Claims 1 to 3, characterised in that nitrobenzene or a dinitrotoluene is used as component b).

5. A process according to Claims 1 to 4, characterised in that tertiary organic amines and/or alkali metal salts of weak acids selected from the group comprising triethylene diamine, potassium acetate and sodium acetate are used as component c).

6. A process according to Claims 1 to 4, characterised in that a tertiary organic amine in combination with a salt of the formula

MeX

wherein

Me represents an alkali metal cation and

X represents an iodide, cyanate or thiocyanate anion, is used as component c).

7. A process according to Claims 1 to 6, characterised in that secondary amines are used as component e).

## Revendications

1. Procédé de production d'uréthannes par réaction de composés aminés aromatiques avec des alcools aliphatiques, cycloaliphatiques ou araliphatiques et l'oxyde de carbone, caractérisé en ce qu'on conduit la réaction en présence

a) de soufre et/ou de sélénium et/ou de composés de ces éléments,

b) de composés aromatiques nitrés,

c) d'amines organiques tertiaires et/ou de sels alcalins d'acides faibles,

d) d'agents oxydants choisis dans le groupe comprenant l'oxygène et des composés inorganiques à action oxydante, contenant de l'oxygène lié chimiquement, de métaux des premier, deuxième et cinquième à huitième sous-groupes du Tableau Périodique des Eléments et, le cas échéant,

e) d'ammoniac et/ou d'amines aliphatiques, araliphatiques, cycloaliphatiques ou hétérocycliques ayant au moins un atome d'hydrogène lié à l'azote d'amine.

2. Procédé suivant la revendication 1, caractérisé en ce qu'on utilise comme composé aminé aromatique l'aniline ou un diaminotoluène et, comme alcool, l'alcool éthylique ou l'alcool méthylique.

3. Procédé suivant les revendications 1 et 2, caractérisé en ce qu'on utilise le soufre élémentaire comme composant a).

4. Procédé suivant les revendications 1 à 3, caractérisé en ce qu'on utilise le nitrobenzène ou un dinitrotoluène comme composant b).

5. Procédé suivant les revendications 1 à 4, caractérisé en ce qu'on utilise des amines organiques tertiaires et/ou des sels alcalins d'acides faibles, choisis dans le groupe comprenant la triéthylènediamine, l'acétate de potassium et l'acétate de sodium, comme composant c).

6. Procédé suivant les revendications 1 à 4, caractérisé en ce qu'on utilise comme composant c) une amine organique tertiaire en association avec un sel de formule :

MeX

dans laquelle :

Me est un cation de métal alcalin et

X est un anion iodure, cyanate ou sulfocyanure.

7. Procédé suivant les revendications 1 à 6, caractérisé en ce qu'on utilise des amines secondaires comme composant e).